# EUROPEAN PATENT SPECIFICATION

(11) **EP 2 838 549 B1**
(45) Date of publication and mention of the grant of the patent: **22.09.2021**
(21) Application number: 13757286.3
(22) Date of filing: 08.03.2013
(51) Int. Cl.: A61K 47/68, A61K 47/60, A61K 38/17, A61K 38/16, A61P 1/16, A61K 38/26, A61P 3/06, A61P 3/10

(54) **PHARMACEUTICAL COMPOSITION FOR THE PREVENTION OR TREATMENT OF NON-ALCOHOLIC FATTY LIVER DISEASE**
ARZNEIMITTELZUSAMMENSETZUNG ZUR PRÄVENTION ODER BEHANDLUNG VON NICHT ALKOHOLISCHER FETTLEBERERKRANKUNG
COMPOSITION PHARMACEUTIQUE DESTINÉE À LA PRÉVENTION OU AU TRAITEMENT DE LA STÉATOSE HÉPATIQUE NON ALCOOLIQUE

(30) Priority: 09.03.2012 KR 20120024632
(43) Date of publication of application: 25.02.2015
(73) Proprietor: Hanmi Science Co., Ltd., Gyeonggi-do 445-813 (KR)
(72) Inventor: LIM, Se Young, Gunsan-si Jeollabuk-do 573-320 (KR); PARK, Sung Hee, Seoul 137-784 (KR); SHIN, Ryoung Ae, Seoul 150-030 (KR); CHOI, In Young, Yongin-si Gyeonggi-do 448-755 (KR); KWON, Se Chang, Seoul 135-506 (KR)
(74) Representative: Mewburn Ellis LLP
(86) International application number: PCT/KR2013/001897
(87) International publication number: WO 2013/133667

(56) References cited:
- WO-A1-2011/109787
- WO-A2-2012/011752
- US-A1- 2010 105 877
- US-A1- 2010 330 108
- US-A1- 2010 330 108
- US-A1- 2011 200 623
- Xiaokun Ding ET AL: "Exendin-4, a glucagon-like protein-1 (GLP-1) receptor agonist, reverses hepatic steatosis inob/ob mice", Hepatology, vol. 43, no. 1, 1 January 2006 (2006-01-01), pages 173-181, XP055325697, US ISSN: 0270-9139, DOI: 10.1002/hep.21006
- Jinmi Lee ET AL: "Exendin-4 Improves Steatohepatitis by Increasing Sirt1 Expression in High-Fat Diet-Induced Obese C57BL/6J Mice", PLoS ONE, vol. 7, no. 2, 17 February 2012 (2012-02-17), page e31394, XP055466587, DOI: 10.1371/journal.pone.0031394

## Description

### [Technical Field]

The present disclosure relates to a pharmaceutical composition including a long-acting insulinotropic peptide conjugate which can be used for the prevention or treatment of non-alcoholic fatty liver disease. In particular, the present disclosure relates to an insulinotropic peptide conjugate in which an insulinotropic peptide, a non-peptidyl polymer, and an immunoglobulin Fc region are covalently linked to each other so as to remarkably increase blood half-life, to effectively prevent triglyceride accumulation, and to a use thereof in the prevention or treatment of non-alcoholic fatty liver disease.

The invention is defined by the claims. Any subject-matter falling outside the scope of the claims is provided for information purposes only.

### [Background Art]

Non-alcoholic fatty liver disease refers to a broad spectrum of diseases ranging from simple steatosis, which is not accompanied by an inflammatory response in a patient with no excessive intake of alcohol, to liver fibrosis and liver cirrhosis, which result from the progression of simple steatosis and exhibit hepatocellular inflammation.

Non-alcoholic fatty liver disease may be categorized into primary and secondary non-alcoholic fatty liver diseases depending on the pathological cause. The primary one is caused by hyperlipidemia, diabetes, obesity or the like which is a characteristic of metabolic syndrome. The secondary one is a result of nutritional causes (sudden body weight loss, starvation, intestinal bypass surgery), various drugs, toxic substances (poisonous mushrooms, bacterial toxins), metabolic causes and other factors.

It is known that the incidence of primary non-alcoholic fatty liver disease in which diabetes and obesity, which are important characteristics of metabolic syndrome, are a primary factor is in about 50% of diabetic patients, about 76% of obesity patients, and most obese diabetic patients (Gupte P et al., 2004). Further, when a liver biopsy is performed on diabetic and obesity patients with an increased level of alanine aminotransferase (ALT), the incidence of steatohepatitis is in the range of 18 to 36% (Braillon A et al., 1985).

Currently, there is no established method for determining the cause of non-alcoholic fatty liver disease. This is because the incidence of non-alcoholic fatty liver disease is associated with a variety of factors such as diabetes, obesity, coronary artery diseases, and lifestyle habits. There are some reports about effects of anti-diabetic or obesity drugs on fatty liver disease. Orlistat, which is used as an oral anti-obesity drug, exhibited histological improvements of the liver in patients with steatohepatitis (Hussein et al., 2007), and metformin exhibited decreases in blood levels of hepatic enzymes and hepatic necrotic inflammation and fibrosis in non-alcoholic fatty liver disease patients with no exhibition of diabetes (Bugianesi et al., 2005). Further, thiazolidinedione (TZD) class drugs, which are PPAR (peroxisome proliferator-activated receptor) agonists, inhibit the accumulation of fat in the liver and muscles, and exhibit direct anti-fibrotic actions on the liver in animal models of non-alcoholic fatty liver diseases (Galli A et al., 2002).

Meanwhile, Glucagon-Like Peptide-1 (GLP-1) is an endogenous peptide present in the body and is a hormone secreted from the intestinal L cells in response to stimulation by nutrients or blood glucose level in the intestine. GLP-1 has a variety of physiological activities including regulation of blood glucose level by stimulating insulin secretion, pancreatic β cell proliferation, inhibition of upper gastrointestinal tract motility, and inhibition of appetite. Recently, GLP-1 receptor expression was found in hepatocytes and GLP-1 shows good effects on the treatment of non-alcoholic fatty liver disease by activation of phosphoinositide-dependent kinase-1 (PDK-1) and protein kinase C-ζ (PKC-ζ) which are major proteins in the insulin signaling pathway via the GLP-1 receptor of hepatocytes (Gupta NA et al., 2010). GLP-1 also functions to reduce fatty acid accumulation or protect hepatocytes from death caused by endoplasmic reticulum stress through activation of both chaperone-mediated autophagy (CMA) and macroautophagy (Sharma S et al., 2011). A recent study reported that GLP-1 promotes hepatic lipid oxidation to prevent hepatic fat accumulation and promotes insulin actions (Svegliati-Baroni G et al., 2011). These many reports suggest that GLP-1 derivative can be an important candidate for the development of a prophylactic and therapeutic agent for non-alcoholic fatty liver disease.

However, the primary obstacle for the use of GLP-1 as a therapeutic agent for non-alcoholic fatty liver is its short blood half-life (maximum half-life: 2 minutes). It is attributed to the loss of the titers of GLP-1 through the cleavage between the 8^{th} amino acid (Ala) and the 9^{th} amino acid (Asp) by a dipeptidyl pepdidase IV (DPP IV) in the body. Therefore, various investigations have been made on a GLP-1 analog having resistance to DPP IV and trials have been made for substitution of Ala⁸ with Gly (Deacon et al., 1998; Burcelin et al., 1999), or with Leu or D-Ala (Xiao et al., 2001), thereby increasing the resistance to DPP IV, while maintaining the activity. The N-terminal amino acid His⁷ of GLP-1 is critical for the GLP-1 activity and serves as a target of DPP IV. Accordingly, US Patent No. 5,545,618 describes that the N-terminus is modified with an alkyl or acyl group and Gallwitz, et al. describes that 7^{th} His was subject to N-methylation, or alpha-methylation, or the entire His is substituted with imidazole to increase the resistance to DPP IV and to maintain physiological activity.

In addition to these modifications, an exendin-4, which is a GLP-1 analog purified from the salivary gland of a gila monster (US Patent No. 5,424,686), has resistance to DPP IV and higher physiological activity than GLP-1. As a result, it had an in-vivo half-life of 2 to 4 hours, a time period that was longer than that of GLP-1. However, with only the method for increasing the resistance to DPP IV, the physiological activity is not sufficiently sustained and, for example, in the case of a commercially available exendin-4 (exenatide) it needs to be injected into a patient twice a day. This frequency is still difficult for patients. The peptide prepared to improve the problem is exendin-4 which is resistant to DPP IV, which has a blood half-life of 2 to 4 hours. Although its blood half-life is longer than that of GLP-1, it also needs to be injected every day.

US 2010/0330108 A1 discloses a composition for treating obesity-related diseases comprising an insulinotropic peptide conjugate, more particularly, to a composition for treating obesity-related diseases comprising a conjugate prepared by covalently linking the insulinotropic peptide with a carrier substance via a non-peptidyl linker, and a method for treating obesity-related diseases by using the same.

Xiaokun Ding et al., Hepatology, vol. 43, no. 1, 1 January 2006, pages 173-181, discloses the treatment of hepatic steatosis in in ob/ob mice using Exendin-4. Ob/ob mice, or their lean littermates, were treated with Exendin-4 [10 µg/kg or 20 µg/kg] for 60 days. Ob/ob mice sustained a reduction in the net weight gained during Exendin-4 treatment. Serum glucose and hepatic steatosis was significantly reduced in Exendin-4 treated ob/ob mice. Exendin-4 improved insulin sensitivity in ob/ob mice, as calculated by the homeostasis model assessment. The measurement of thiobarbituric reactive substances as a marker of oxidative stress was significantly reduced in ob/ob-treated mice with Exendin-4. Finally, GLP-1-treated hepatocytes resulted in a significant increase in cAMP production as well as reduction in mRNA expression of stearoyl-CoA desaturase 1 and genes associated with fatty add synthesis; the converse was true for genes associated with fatty add oxidation.

Jinmi Lee et al., PLoS ONE, vol. 7, no. 2, 17 February 2012 (2012-02-17), page e31394, disusses whether the beneficial effects of exendin-4 treatment on fatty liver are mediated via Sirt1 in high-fat (HF) diet-induced obese C578L/6J mice and related cell culture models. Exendin-4 treatment decreased body weight, serum free fatty acid {FA), and triglyceride levels in HF-induced obese C578U6J mice. Histological analysis showed that exendin-4 reversed HF-induced hepatic accumulation of lipids and inflammation. Exendin-4 treatment increased mRNA and protein expression of Sirt1 and its downstream factor, AMPK, in vivo and also induced genes associated with FA oxidation and glucose metabolism. In addition, a significant increase in the hepatic expression of Lkb1 and Nampt mRNA was observed in exendin-4-treated groups. Increased expression of phospho-Foxol and GLUT2, which are involved in hepatic glucose metabolism, was also observed. In HepG2 and Huh7 cells, mRNA and protein expressions of GLP-1 R were increased by exendln-4 treatment in a dose-dependent manner. Exendin-4 enhanced protein expression of Slrt1 and phospho-AMPKα in HepG2 cells treated with 0.4 mM palmitic acid. It was also found that Sirt1 was an upstream regulator of AMPK in hepatocytes. A finding of this study was the observation that expression of GLP-1 R is proportional to exendin-4 concentration and exendin-4 could attenuate fatty liver through activation of Sirt1.

US 2010/0105877 A1 discloses an insulinotropic peptide conjugate having altered in-vivo duration of efficacy and stability, comprising an insulinotropic peptide, a non-peptide polymer and an immunoglobulin Fc region, which are covalently linked to each other, and a use of the same. The insulinotropic peptide conjugate has the in-vivo activity which is maintained relatively high, and has increased blood half-life.

### [Disclosure]

### [Technical Problem]

Accordingly, the present authors used a method of site-specifically linking an immunoglobulin Fc region, a non-peptidyl polymer, and an insulinotropic peptide by a covalent bond so as to maximize the effects of increasing the blood half-life of the insulinotropic peptide and maintaining the in-vivo activity. As a result, the present authors found that the method remarkably increased the blood half-life of the peptide conjugate and provided much longer blood half-life than the known in-frame fusion method. The present authors also found that the conjugate prepared by site-specific linkage of the immunoglobulin Fc to an amine group or a thiol group present at an amino acid residue other than the N-terminus of the insulinotropic peptide maintains higher titers than a conjugate prepared by linkage at the N-terminus of the insulinotropic peptide. Consequently, it was confirmed that the conjugate shows excellent therapeutic effects on non-alcoholic fatty liver disease even though it is less frequently administered than the known exendin-4 formulations, thereby completing the present disclosure.

### [Technical Solution]

An object of the present disclosure is to provide a long-acting insulinotropic peptide conjugate which maintains a prolonged in-vivo half-life and effectively prevents triglyceride accumulation and thus is useful for the prevention or treatment of non-alcoholic fatty liver disease.

### [Advantageous Effects]

The insulinotropic peptide conjugate, according to the present disclosure, maintains in-vivo activity of the peptide at a relatively high level, has a remarkably increased blood half-life, and effectively activates major proteins involved in lipolysis to prevent triglyceride accumulation, thereby being useful for the prevention and treatment of non-alcoholic fatty liver disease.

### [Description of Drawings]

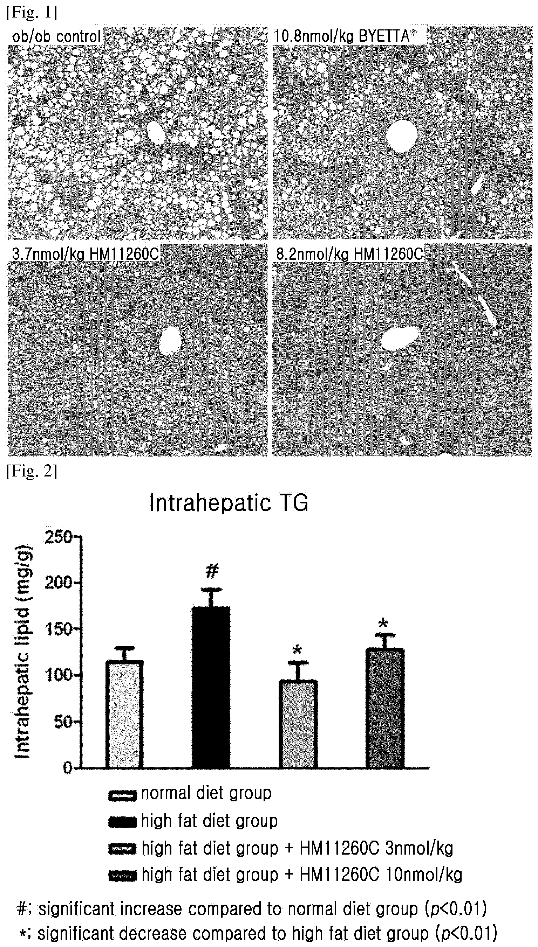

FIG. 1 shows images of the liver tissue of ob/ob mouse which was administered with the long-acting exendin-4 conjugate according to one embodiment of the present disclosure (Hematoxylin & Eosin staining, H&E staining, area stained in purple: normal liver tissue, area stained in white: lipid droplet); and

FIG. 2 shows a graph of intrahepatic triglyceride accumulation in high fat induced-obese mice which were administered with the long-acting exendin-4 conjugate according to one embodiment of the present disclosure (#: a significant increase at 99% confidence, compared to a normal diet group (p<0.01), *: a significant decrease at 99% confidence, compared to a high fat diet group (p<0.01)).

### [Best Mode]

In one aspect, to achieve the above objects, one embodiment of the disclosure relates to a pharmaceutical composition for the prevention or treatment of non-alcoholic fatty liver disease including an insulinotropic peptide drug conjugate, which is prepared by covalently linking an insulinotropic peptide and an immunoglobulin Fc region via a non-peptidyl polymer, as an active ingredient.

In the pharmaceutical composition of the present disclosure, the insulinotropic peptide is selected from the group consisting of exendin-4, an exendin-4 derivative prepared by deleting the N-terminal amine group of exendin-4, an exendin-4 derivative prepared by substituting the N-terminal amine group of exendin-4 with a hydroxyl group, an exendin-4 derivative prepared by modifying the N-terminal amine group of exendin-4 with a dimethyl group, and an exendin-4 derivative prepared by deleting alpha-carbon of the N-terminal histidine residue of exendin-4 and the N-terminal amine group linked to the alpha-carbon.

The non-peptidyl polymer is selected from the group consisting of polyethylene glycol, polypropylene glycol, copolymers of ethylene glycol-propylene glycol, polyoxyethylated polyols, polyvinyl alcohol, polysaccharides, dextran, polyvinyl ethyl ether, biodegradable polymers, lipid polymers, chitins, hyaluronic acid, and combinations thereof.

The insulinotropic peptide of the present disclosure is a peptide possessing an insulinotropic function to promote the synthesis and the expression of insulin in a pancreatic beta cell. These peptides include precursors, derivatives, fragments, variants or the like, and preferably GLP (glucagon like peptide)-1, exendin-3, exendin-4 or the like.

GLP-1 is a hormone that is secreted by the small intestine. In general, it promotes the biosynthesis and secretion of insulin, inhibits the secretion of glucagon, and promotes glucose absorption in the cells. In the small intestine, a glucagon precursor is decomposed into three peptides, that is, glucagon, GLP-1, and GLP-2. Here, the GLP-1 means GLP-1 (1-37), which is originally in the form having no insulinotropic function. But it is then processed and converted into the activated GLP-1 (7-37) form. The amino acid sequence of GLP-1 (7-37) is as follows:
GLP-1(7-37)
HAEGT FTSDV SSYLE GQAAK EPIAW LVKGR G

The GLP-1 derivative means a peptide which exhibits an amino acid sequence homology of at least 80% with that of GLP-1, may be in the chemically modified form, and exhibits an insulinotropic function of at least equivalent to or more than that of GLP-1.

The GLP-1 fragment means the form in which one or more amino acids are added or deleted at the N-terminus or C-terminus of the native GLP-1, and the added amino acid is possibly a non-naturally occurring amino acid (e.g., D-type amino acid).

The GLP-1 variant means a peptide possessing an insulinotropic function which has one or more amino acid sequences different from those of the native GLP-1.

The exendin-3 and the exendin-4 are insulinotropic peptides consisting of 39 amino acids which have a 53% amino acid sequence homology with GLP-1. The amino acid sequences of the exendin-3 and the exendin-4 are as follows:
Exendin-3
   HSDGT FTSDL SKQME EEAVR LFIEW LKNGG PSSGA PPPS
Exendin-4
   HGEGT FTSDL SKQME EEAVR LFIEW LKNGG PSSGA PPPS

The exendin derivative means a peptide having at least 80% amino acid sequence homology with the native exendin, which may have some groups on the amino acid residue chemically substituted, and exhibits an insulinotropic function of at least equivalent to or more than that of the native exendin.

The exendin fragment means a fragment having one or more amino acids added or deleted at the N-terminus or the C-terminus of the native exendin, and the added amino acid is possibly a non-naturally occurring amino acid (e.g., D-type amino acid).

The exendin variant means a peptide possessing an insulinotropic function which has one or more amino acid sequences different from those of the native exendin.

In a specific embodiment, the native insulinotropic peptide used in the present disclosure and the modified insulinotropic peptide may be synthesized using a solid phase synthesis method and most of the native peptides, including the native insulinotropic peptide, may be produced by a recombination technology.

Further, the insulinotropic peptide used in the present disclosure may bind to the non-peptidyl polymer on various sites.

The conjugate prepared in the present disclosure may have activity which varies depending on the binding sites of the insulinotropic peptide.

For example, it may be coupled with the N-terminus, and other terminus including the C-terminus, respectively, which indicates difference in the in-vitro activity. The aldehyde reactive group selectively binds to the N-terminus at a low pH and may bind to a lysine residue to form a covalent bond at a high pH, for example, pH 9.0. A pegylation reaction is allowed to proceed with varying pH and an ion exchange column may then be used to separate a positional isomer from the reaction mixture.

If the insulinotropic peptide is to be coupled at a site other than the N-terminus, which is an important site for the in-vivo activity, a reactive thiol group can be introduced to the site of amino acid residue to be modified in the native amino acid sequence so as to form a covalent bond using a maleimide linker at the non-peptidyl polymer.

If the insulinotropic peptide is to be coupled at a site other than the N-terminus, which is an important site for the in-vivo activity, a reactive amine group can be introduced to the site of amino acid residue to be modified in the native amino acid sequence so as to form a covalent bond using an aldehyde linker at the non-peptidyl polymer.

When the aldehyde linker at the non-peptidyl polymer is used, it is reacted with an amine group at the N-terminus and the lysine residue, and a modified form of the insulinotropic peptide may be used to selectively increase the reaction yield. For example, only one amine group to be reacted may be retained on a desired site, using an N-terminus blocking method, a lysine residue substituting method, a method for introducing an amine group at a carboxyl terminus, or the like, thereby increasing the yield of pegylation and coupling reactions. The methods for protecting the N-terminus include dimethylation, as well as methylation, deamination, acetylation, etc., but are not limited to such alkylation methods.

In one preferred embodiment of the disclosure, the insulinotropic peptide conjugate of the present disclosure is an insulinotropic peptide conjugate in which an immunoglobulin Fc region specifically binds to an amine group other than ones at the N-terminus of the insulinotropic peptide.

In one specific embodiment, the present authors induced a pegylation of a native exendin-4 at pH 9.0 to selectively couple the PEG to the lysine residue of the insulinotropic peptide. Alternatively, the exendin-4 derivatives having the N-terminus deleted or protected may be synthesized to be coupled. The pegylation at the N-terminus can be blocked either by deleting the alpha amine group of the N-terminal histidine or by modifying the N-terminal histidine with two methyl groups. Such N-terminal modification does not influence in-vitro activity (Table 1).

Unlike the N-terminal coupling of exendin-4, coupling at the lysine residue maintained the in-vitro activity at approximately 6% (Table 1). Further, the exendin-4-PEG-immunoglobulin Fc conjugate prepared in the present disclosure exhibited a remarkably increased blood half-life of 60∼70 hours, indicating an unexpectedly high duration of efficacy. Therefore, the titer reduction was also minimized by coupling to the lysine residue which does not affect the activity, and thus a new long-acting exendin-4 formulation capable of maintaining its in-vivo activity could be prepared.

The immunoglobulin Fc region is safe for use as a drug carrier because it is a biodegradable polypeptide that is metabolized in vivo. Also, the immunoglobulin Fc region has a relatively low molecular weight as compared to the whole immunoglobulin molecules and thus it is advantageous in the preparation, purification, and yield of the conjugate. Since the immunoglobulin Fc region does not contain a Fab fragment whose amino acid sequence differs according to the antibody subclasses and which thus is highly non-homogenous, it can be expected that the immunoglobulin Fc region may greatly increase the homogeneity of substances and be less antigenic.

The term "immunoglobulin Fc region" as used herein refers to the heavy-chain constant region 2 (C_{H}2) and the heavy-chain constant region 3 (C_{H}3) and excludes the variable regions of the heavy and light chains, the heavy-chain constant region 1 (C_{H}1), and the light-chain constant region 1 (C_{L}1) of the immunoglobulin. It may further include a hinge region at the heavy-chain constant region. Also, the immunoglobulin Fc region of the present disclosure may contain a part or all of the Fc region including the heavy-chain constant region 1 (C_{H}1) and/or the light-chain constant region 1 (C_{L}1), except for the variable regions of the heavy and light chains, as long as it has effects substantially similar to or better than the native protein. Also, the immunoglobulin Fc region may be a fragment having a deletion in a relatively long portion of the amino acid sequence of C_{H}2 and/or C_{H}3. That is, the immunoglobulin Fc region of the present disclosure may include 1) a C_{H}1 domain, a C_{H}2 domain, a C_{H}3 domain and a C_{H}4 domain, 2) a C_{H}1 domain and a C_{H}2 domain, 3) a C_{H}1 domain and a C_{H}3 domain, 4) a C_{H}2 domain and a C_{H}3 domain, 5) a combination of one or more domains and an immunoglobulin hinge region (or a portion of the hinge region), and 6) a dimer of each domain of the heavy-chain constant regions and the light-chain constant region.

The immunoglobulin Fc region of the present disclosure includes a native amino acid sequence and a sequence derivative (mutant) thereof. An amino acid sequence derivative is a sequence that is different from the native amino acid sequence due to a deletion, an insertion, a non-conservative or conservative substitution, or combinations thereof of one or more amino acid residues. For example, in an IgG Fc, amino acid residues known to be important in binding at positions 214 to 238, 297 to 299, 318 to 322, or 327 to 331 may be used as a suitable target for modification. Also, other various derivatives are possible, including one in which a region capable of forming a disulfide bond is deleted or certain amino acid residues are eliminated at the N-terminus of a native Fc form, or a methionine residue is added thereto. Further, to remove effector functions, a deletion may occur in a complement-binding site, such as a C1q-binding site and an ADCC site. Techniques of preparing such sequence derivatives of the immunoglobulin Fc region are disclosed in International Patent Publication Nos. WO 97/34631 and WO 96/32478.

Amino acid exchanges in proteins and peptides, which do not generally alter the activity of molecules, are known in the art (H.Neurath, R.L.Hill, The Proteins, Academic Press, New York, 197 9). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly in both directions.

The Fc region, if desired, may be modified by phosphorylation, sulfation, acrylation, glycosylation, methylation, farnesylation, acetylation, amidation, and the like.

The aforementioned Fc derivatives are derivatives that have a biological activity identical to the Fc region of the present disclosure or improved structural stability against heat, pH, or the like.

In addition, these Fc regions may be obtained from native forms isolated from humans and other animals including cows, goats, swine, mice, rabbits, hamsters, rats and guinea pigs, or may be recombinants or derivatives thereof, obtained from transformed animal cells or microorganisms. Herein, they may be obtained from a native immunoglobulin by isolating whole immunoglobulins from human or animal organisms and treating them with a proteolytic enzyme. Papain digests the native immunoglobulin into Fab and Fc regions, and pepsin treatment results in the production of pF'c and F(ab)2 fragments. These fragments may be subjected to size exclusion chromatography to isolate Fc or pF'c.

Preferably, a human-derived Fc region is a recombinant immunoglobulin Fc region that is obtained from a microorganism.

In addition, the immunoglobulin Fc region may be in the form of having native sugar chains, increased sugar chains compared to a native form, or decreased sugar chains compared to the native form, or may be in a deglycosylated form. The increase, decrease or removal of the immunoglobulin Fc sugar chains may be achieved by methods common in the art, such as a chemical method, an enzymatic method and a genetic engineering method using a microorganism. The removal of sugar chains from an Fc region results in a sharp decrease in binding affinity to the complement (clq) and a decrease or loss in antibody-dependent cell-mediated cytotoxicity or complement-dependent cytotoxicity, thereby not inducing unnecessary immune responses in-vivo. In this regard, an immunoglobulin Fc region in a deglycosylated or aglycosylated form may be more suitable to the object of the present disclosure as a drug carrier.

As used herein, the term "deglycosylation" refers to enzymatically removed sugar moieties from an Fc region and the term "aglycosylation" means that an Fc region is produced in an unglycosylated form by a prokaryote, preferably E. coli.

While the immunoglobulin Fc region may preferably be derived from humans it may also be derived from other animals including cows, goats, swine, mice, rabbits, hamsters, rats and guinea pigs. In addition, the immunoglobulin Fc region may be an Fc region that is derived from IgG, IgA, IgD, IgE and IgM, or that is made by combinations thereof or hybrids thereof. Preferably, it is derived from IgG or IgM, which is among the most abundant proteins in human blood, and most preferably derived from IgG, which is known to enhance the half-lives of ligand-binding proteins.

On the other hand, the term "combination", as used herein, means that polypeptides encoding single-chain immunoglobulin Fc regions of the same origin are linked to a single-chain polypeptide of a different origin to form a dimer or multimer. That is, a dimer or multimer may be formed from two or more fragments selected from the group consisting of IgG Fc, IgA Fc, IgM Fc, IgD Fc, and IgE Fc fragments.

The term "hybrid", as used herein, means that sequences encoding two or more immunoglobulin Fc regions of different origin are present in a single-chain immunoglobulin Fc region. In the present disclosure, various types of hybrids are possible. That is, domain hybrids may be composed of one to four domains selected from the group consisting of CH1, CH2, CH3 and CH4 of IgG Fc, IgM Fc, IgA Fc, IgE Fc and IgD Fc, and may include the hinge region.

On the other hand, IgG may be divided into IgG1, IgG2, IgG3 and IgG4 subclasses, and the present disclosure may include combinations and hybrids thereof. Preferred are IgG2 and IgG4 subclasses, and most preferred is the Fc region of IgG4 rarely having effector functions such as CDC (complement dependent cytotoxicity).

That is, as the drug carrier of the present disclosure, the most preferable immunoglobulin Fc region is a human IgG4-derived non-glycosylated Fc region. The human-derived Fc region is more preferable than a non-human derived Fc region which may act as an antigen in the human body and cause undesirable immune responses such as the production of a new antibody against the antigen.

The term "non-peptidyl polymer", as used herein, refers to a biocompatible polymer including two or more repeating units linked to each other by any covalent bond excluding a peptide bond.

The non-peptidyl polymer which can be used in the present disclosure may be selected from the group consisting of polyethylene glycol, polypropylene glycol, copolymers of ethylene glycol and propylene glycol, polyoxyethylated polyols, polyvinyl alcohol, polysaccharides, dextran, polyvinyl ethyl ether, biodegradable polymers such as PLA (polylactic acid) and PLGA (polylactic-glycolic acid), lipid polymers, chitins, hyaluronic acid, and combinations thereof, the preferred of which is polyethylene glycol. Also, derivatives thereof well known in the art and being easily prepared within the skill of the art are included in the scope of the present disclosure.

The peptide linker which is used in the fusion protein obtained by a conventional in-frame fusion method has drawbacks in that it is easily in-vivo cleaved by a proteolytic enzyme and thus a sufficient effect of increasing the blood half-life of the active drug by a carrier cannot be obtained as expected. However, in the present disclosure, a polymer having resistance to the proteolytic enzyme can be used to maintain the blood half-life of the peptide to be similar to that of the carrier. Therefore, any non-peptidyl polymer to be used in the present disclosure can be used without any limitation as long as it is a polymer having the aforementioned function, that is, a polymer having resistance to the in-vivo proteolytic enzyme. The non-peptidyl polymer preferably has a molecular weight in the range of 1 to 100 kDa, and preferably of 1 to 20 kDa. Also, the non-peptidyl polymer of the present disclosure, linked to the immunoglobulin Fc region, may be one polymer or a combination of different types of polymers.

The non-peptidyl polymer used in the present disclosure has a reactive group capable of binding to the immunoglobulin Fc region and the protein drug.

The non-peptidyl polymer has a reactive group at both ends which is preferably selected from the group consisting of a reactive aldehyde group, a propionaldehyde group, a butyraldehyde group, a maleimide group and a succinimide derivative. The succinimide derivative may be succinimidyl propionate, hydroxy succinimidyl, succinimidyl carboxymethyl, or succinimidyl carbonate. In particular, when the non-peptidyl polymer has a reactive aldehyde group at both ends, it is effective in linking at both ends with a physiologically active polypeptide and an immunoglobulin with minimal non-specific reactions. A final product generated by reductive alkylation via an aldehyde bond is much more stable than when linked by an amide bond. The aldehyde reactive group selectively binds to the N-terminus at a low pH and can bind to a lysine residue to form a covalent bond at a high pH, for example, at pH 9.0.

The reactive groups at both ends of the non-peptidyl polymer may be the same or different. For example, the non-peptide polymer may possess a maleimide group at one end and at the other end it may possess an aldehyde group, a propionaldehyde group or a butyraldehyde group. When a polyethylene glycol having a reactive hydroxy group at both ends thereof is used as the non-peptidyl polymer, the hydroxy group may be activated to various reactive groups by known chemical reactions, or a polyethylene glycol having a commercially available modified reactive group may be used so as to prepare the insulinotropic peptide conjugate of the present disclosure.

In another embodiment, the present disclosure provides a method for preparing an insulinotropic peptide conjugate including the steps of:
(1) covalently linking a non-peptidyl polymer having a reactive group of aldehyde, maleimide, or succinimide derivative at both ends thereof, with an amine group or thiol group of an insulinotropic peptide;
(2) isolating a conjugate including the insulinotropic peptide from the reaction mixture of (1), in which the non-peptidyl polymer is covalently linked to a site other than the amino terminus; and
(3) covalently linking an immunoglobulin Fc region to the other end of the non-peptidyl polymer of the isolated conjugate so as to produce a peptide conjugate having the immunoglobulin Fc region and the insulinotropic peptide, which are linked to each end of the non-peptidyl polymer.

The term "conjugate", as used herein, refers to an intermediate prepared by covalently linking the non-peptidyl polymer with the insulinotropic peptide and subsequently the immunoglobulin Fc region is linked to the other end of the non-peptidyl polymer in the conjugate.

In one preferred embodiment, the present disclosure provides a preparation method including the steps of:
(1) covalently linking a non-peptidyl polymer having an aldehyde reactive group at both ends thereof with the lysine residue of exendin-4;
(2) isolating a conjugate including exendin-4 from the reaction mixture of (1), in which the non-peptidyl polymer is covalently linked to the lysine residue; and
(3) covalently linking an immunoglobulin Fc region to the other end of the non-peptidyl polymer of the isolated conjugate so as to produce a protein conjugate including the immunoglobulin Fc region and exendin-4, which are linked to each end of the non-peptidyl polymer. More preferably, the non-peptidyl polymer and the lysine residue of exendin-4 in (1) are linked at pH 9.0 or higher.

The insulinotropic peptide conjugate of the present disclosure activates major proteins of the insulin signaling pathway via the GLP-1 receptor, and thus can be used for the prevention or treatment of non-alcoholic fatty liver disease. In particular, the insulinotropic peptide conjugate of the present disclosure increases the activity of PKC-ζ (Protein Kinase C-ζ) which regulates enzymatic activity involved in lipolysis and maintains in-vivo activity of the known insulinotropic peptide which increases Glut2 (Glucose transporter protein-2) expression, and increases the blood half-life of insulinotropic peptide, thereby remarkably increasing duration of in-vivo efficacy. Accordingly, excellent therapeutic effects on non-alcoholic fatty liver disease can be obtained with less administration frequency than the known formulations.

In the present disclosure, non-alcoholic fatty liver disease (NAFLD) includes primary and secondary non-alcoholic fatty liver diseases, and more specifically means non-alcoholic fatty liver disease caused by primary hyperlipidemia, diabetes, or obesity. For example, non-alcoholic fatty liver disease includes simple steatosis, fatty liver diseases caused by malnutrition, starvation, obesity and diabetes, steatohepatitis, and liver fibrosis and liver cirrhosis occurring due to the progression of these diseases.

The pharmaceutical composition including the insulinotropic peptide conjugate of the present disclosure may further include a pharmaceutically acceptable carrier. For oral administration, the pharmaceutically acceptable carrier may include a binder, a lubricant, a disintegrator, an excipient, a solubilizer, a dispersing agent, a stabilizer, a suspending agent, a coloring agent, and a perfume. For injectable preparations, the pharmaceutically acceptable carrier may include a buffering agent, a preserving agent, an analgesic, a solubilizer, an isotonic agent, and a stabilizer. For preparations for topical administration, the pharmaceutically acceptable carrier may include a base, an excipient, a lubricant, and a preserving agent. The pharmaceutical composition of the present disclosure may be formulated into a variety of dosage forms in combination with the aforementioned pharmaceutically acceptable carriers. For example, for oral administration, the pharmaceutical composition may be formulated into tablets, troches, capsules, elixirs, suspensions, syrups or wafers. For injectable preparations, the pharmaceutical composition may be formulated into an ampule as a single-dose dosage form or a unit dosage form, such as a multidose container. The pharmaceutical composition may be also formulated into solutions, suspensions, tablets, pills, capsules and long-acting preparations.

On the other hand, examples of the carrier, the excipient, and the diluent suitable for the pharmaceutical formulations include lactose, dextrose, sucrose, sorbitol, mannitol, xylitol, erythritol, maltitol, starch, acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oils. In addition, the pharmaceutical formulations may further include fillers, anti-coagulating agents, lubricants, humectants, perfumes, and antiseptics.

The conjugate according to the present disclosure is useful to prevent or treat non-alcoholic fatty liver disease. Accordingly, a pharmaceutical composition including the conjugate may be administered for the treatment of the disease.

The term "administration", as used herein, means introduction of a predetermined substance into a patient by a certain suitable method. The conjugate of the present disclosure may be administered via any of the common routes as long as it is able to reach a desired tissue. A variety of modes of administration are contemplated, including intraperitoneally, intravenously, intramuscularly, subcutaneously, intradermally, orally, topically, intranasally, intrapulmonarily and intrarectally, but the present disclosure is not limited to these exemplified modes of administration. However, since peptides are digested upon oral administration, active ingredients of a composition for oral administration should be coated or formulated for protection against degradation in the stomach. Preferably, the present composition may be administered in an injectable form. In addition, the pharmaceutical composition may be administered using a certain apparatus capable of transporting the active ingredients into a target cell.

The pharmaceutical composition of the present disclosure can be determined by several related factors including the types of diseases to be treated, administration routes, the patient's age, gender, weight and severity of the illness, as well as by the types of the drug as an active component. Since the pharmaceutical composition of the present disclosure has excellent duration of in-vivo efficacy and titer, it can remarkably reduce the administration frequency and dose of pharmaceutical drugs of the present disclosure.

Further, the pharmaceutical composition of the present disclosure may be used singly or in combination with surgical operation, hormone therapy, drug therapy and biological response regulators in order to prevent and treat non-alcoholic fatty liver disease.

In one aspect of the present disclosure relates to a use of the pharmaceutical composition in the preparation of drugs for the prevention or treatment of non-alcoholic liver disease.

### [Mode]

Hereinafter, the present disclosure will be described in more detail with reference to the following Examples. However, these Examples are for illustrative purposes only, and the disclosure is not intended to be limited by these Examples.

### Example 1. Test of in-vitro activity of long-acting exendin-4

A variety of long-acting exendin-4 derivatives used in this experiment were prepared in the same manner as in Korean Patent No. 10-1058315 of the present authors.

A method for measuring the in-vitro cell activity was used so as to measure the efficacy of long acting preparation of exendin-4. In the in-vitro activity measurement, RIN-m5F was used, which is known as a rat insulinoma cell. Because this cell has a GLP-1 receptor, it is commonly used in the methods for measuring the in-vitro activity of the GLP-1 family. RIN-m5F was treated with GLP-1, exendin-4, and test materials at varying concentrations. EC50 values were determined by measuring the occurrence of cAMP's, which are signaling molecules in the cells, caused by the test materials, and compared to each other. The results are summarized in Table 1.

**[Table 1]**

| Test material | Blood half-life (hr) | In-vitro titer (%) |
|---|---|---|
| Exendin-4 | 0.7 | 100 |
| Exendin-4(N)-PEG-Fc | 61.5 | < 0.2 |
| Exendin-4(Lys27)-PEG-Fc | 70.5 | 6.3 |

| | | |
|---|---|---|
| Exendin-4(N)-PEG-Fc: conjugate prepared by linking the N-terminus of exendin-4 and Fc region via PEG. Exendin-4(Lys27)-PEG-Fc: conjugate prepared by linking the 27^{th} lysine residue of exendin-4 and Fc region via PEG. | | |

As shown in Table 1, when the non-peptidyl polymer was linked to the lysine residue other than the N-terminus of the native exendin-4, the in-vitro titer was maintained at 6.3%, and the blood half-life was remarkably increased to approximately 70 hours.

### Example 2. Effects on fatty liver formation in obese animal model ob/ob mouse

### <2-1> Division of experimental animals

Female 5-week-old ob/ob mice (C57BL/6JHamSlc-ob/ob, 24-34 g) were purchased from Slc, Japan. The ob/ob mouse is an animal model commonly used in the efficacy tests of anti-obesity and anti-diabetic formulations. They were freely fed with solid feed for experimental animals, which was sterilized by radiation (manufacturer: Picolab Rodent Diet, product name: 5053), and had free access to filtered, UV irradiation-sterilized tap water in a water bottle. They were maintained in a casing system meeting the GLP Standard requirements on a 12 hr dark-light cycle (light switched on at 6:00 am and off at 6:00 pm) in accordance with animal care standard guidelines. Thereafter, healthy ob/ob mice were selected and acclimated to the laboratory conditions for 1 week. Then, drug administration was performed, and mice were divided into 4 groups and administered as follows.
Group 1 (negative control): subcutaneous injection of DULBECCO'S PHOSPHATE BUFFERED SALINE (Sigma) once or more a week at an administration volume of 5 ml/kg
Group 2 (positive control): subcutaneous injection of 10.8 nmol/kg of BYETTA every day at an administration volume of 5 ml/kg
Group 3 (3.7 nmol/kg of long-acting exendin-4 derivative-treated group): subcutaneous injection of 3.7 nmol/kg of long-acting exendin-4 derivative (HM11260C) once a week at an administration volume of 5 ml/kg
Group 4 (8.2 nmol/kg of long-acting exendin-4 derivative-treated group): subcutaneous injection of 8.2 nmol/kg of long-acting exendin-4 derivative (HM11260C) once a week at an administration volume of 5 ml/kg

BYETTA (Eli Lilly) is the native exendin-4, and the long-acting exendin-4 derivative (HM11260C) is a CA exendin-4-PEG-Fc conjugate prepared by linking imidazoacetyl-exendin-4 with removal of alpha carbon of the first amino acid histidine to Fc region via PEG, described in Korean Patent No. 10-1058315.

Each group was administered with a saline solution or drugs for 7 weeks, and their effects on fatty liver formation were analyzed.

### <2-2> Effects of long-acting exendin-4 derivative on fatty liver formation

In order to examine the effects of the long-acting exendin-4 derivatives according to the present disclosure on fatty liver formation in ob/ob mouse, the following experiment was performed. Drugs were administered into the groups divided in Example <2-1>, and the livers were taken from the ob/ob mice, and a part thereof was fixed in 4% formaldehyde and embedded in paraffin, followed by H&E staining. The results are shown in FIG. 1.

As shown in FIG. 1, pathological features of fatty liver were clearly observed in the negative control group treated with a vehicle, whereas a remarkable dose-dependent reduction in pathological features of fatty liver was observed in the experimental group treated with the long-acting exendin-4 derivative of the present disclosure. It was also found that the long-acting exendin-4 derivative of the present disclosure showed excellent therapeutic effects on fatty liver even with a lower dose, compared to the positive control BYETTA.

### Example 3. Effects on intrahepatic triglyceride accumulation in high fat induced-obese mice

### <3-1> Division of experimental animals

6-week-old C57BL/6 mice were stabilized and divided into two groups, and received a normal diet containing 10% fat and a high-fat diet containing 60% fat for 12 weeks, (manufacturer: Research diets Inc., product name: D12492). Thus, normal mice and high fat induced-obese mice were prepared and used for experiments. They were maintained in a casing system meeting the GLP Standard requirements on a 12 hr dark-light cycle (light switched on at 6:00am and off at 6:00pm) in accordance with animal care standard guidelines. Thereafter, healthy high fat induced-obese mice were selected and acclimated to the laboratory conditions for 1 week. Then, drug administration was performed, and mice were divided into 4 groups and administered as follows.
Group 1 (normal diet group): subcutaneous injection of DULBECCO'S PHOSPHATE BUFFERED SALINE (Sigma) once or more a week at an administration volume of 5 ml/kg
Group 2 (high fat diet group): subcutaneous injection of DULBECCO'S PHOSPHATE BUFFERED SALINE (Sigma) once or more a week at an administration volume of 5 ml/kg
Group 3 (high fat diet group treated with 3 nmol/kg of long-acting exendin-4 derivative): subcutaneous injection of 3 nmol/kg of long-acting exendin-4 derivative (HM11260C) once a week at an administration volume of 5 ml/kg
Group 4 (high fat diet group treated with 10 nmol/kg of long-acting exendin-4 derivative): subcutaneous injection of 10 nmol/kg of long-acting exendin-4 derivative (HM11260C) once a week at an administration volume of 5 ml/kg

Each group was administered with a saline solution or drugs for 2 weeks, and the amount of triglyceride accumulated in the liver tissue was analyzed.

### <3-2> Measurement of intrahepatic triglyceride accumulation in high fat induced-obese mice

The livers were taken from the groups divided in Example <3-1>, which were high fat induced-obese mice administered with or without the long-acting exendin-4 derivative, and intrahepatic triglyceride concentrations were determined. As shown in FIG. 2, intrahepatic triglyceride concentration of the high fat diet group was 172.3 mg/g, which was higher than that of the low fat diet group (114.0 mg/g), but the high fat diet group treated with 3 nmol/kg of the long-acting exendin-4 derivative showed 93 mg/g of triglyceride level, showing a 46% reduction, compared to the high fat diet group. These results suggest that the long-acting exendin-4 derivative of the present disclosure has therapeutic effects on non-alcoholic fatty liver disease.
## Claims

1. A pharmaceutical composition for use in the prevention or treatment of non-alcoholic fatty liver disease comprising an insulinotropic peptide drug conjugate prepared by covalently linking an insulinotropic peptide and an immunoglobulin Fc region via a non-peptidyl polymer as an active ingredient, wherein the insulinotropic peptide is selected from the group consisting of exendin-4, an exendin-4 derivative prepared by deleting the N-terminal amine group of exendin-4, an exendin-4 derivative prepared by substituting the N-terminal amine group of exendin-4 with a hydroxyl group, an exendin-4 derivative prepared by modifying the N-terminal amine group of exendin-4 with a dimethyl group, and an exendin-4 derivative prepared by deleting alpha-carbon of the N-terminal histidine residue of exendin-4 and the N-terminal amine group linked to the alpha-carbon, and
the non-peptidyl polymer is selected from the group consisting of polyethylene glycol, polypropylene glycol, copolymers of ethylene glycol-propylene glycol, polyoxyethylated polyols, polyvinyl alcohol, polysaccharides, dextran, polyvinyl ethyl ether, biodegradable polymers, lipid polymers, chitins, hyaluronic acid, and combinations thereof, wherein the non-peptidyl polymer is linked to a lysine residue at position 27 of the insulinotropic peptide; and wherein the non-alcoholic fatty liver disease is selected from the group consisting of simple steatosis, fatty liver diseases caused by starvation, liver fibrosis and liver cirrhosis.

2. The pharmaceutical composition for use according to claim 1, wherein the immunoglobulin Fc region and the insulinotropic peptide are linked at both ends of the non-peptidyl polymer, respectively.

3. The pharmaceutical composition for use according to claim 1, wherein the non-peptidyl polymer is polyethylene glycol.

4. The pharmaceutical composition for use according to claim 1, wherein the immunoglobulin Fc region is aglycosylated.

5. The pharmaceutical composition for use according to claim 1, wherein the immunoglobulin Fc region is composed of one to four domains selected from the group consisting of CH1, CH2, CH3 and CH4 domains.

6. The pharmaceutical composition for use according to claim 5, wherein the immunoglobulin Fc region further include a hinge region.

7. The pharmaceutical composition for use according to claim 1, wherein the immunoglobulin Fc region is an Fc region that is derived from an immunoglobulin selected from the group consisting of IgG, IgA, IgD, IgE and IgM, optionally wherein the immunoglobulin Fc region is an IgG4 Fc region, and optionally wherein the immunoglobulin Fc region is a human non-glycosylated IgG4 Fc region.

8. The pharmaceutical composition for use according to claim 1, wherein the reactive group of the non-peptidyl polymer is selected from the group consisting of an aldehyde group, a propionaldehyde group, a butyraldehyde group, a maleimide group and a succinimide derivative.

9. The pharmaceutical composition for use according to claim 8, wherein the succinimide derivative is selected from the group consisting of succinimidyl propionate, succinimidyl carboxymethyl, hydroxy succinimidyl, and succinimidyl carbonate.

10. The pharmaceutical composition for use according to claim 1, wherein the non-peptidyl polymer has reactive aldehyde groups at both ends thereof.

11. The pharmaceutical composition for use according to claim 1, wherein the insulinotropic peptide drug conjugate increases the activity of PKC-ζ (Protein Kinase C-ζ) regulating the enzymatic activity involved in lipolysis.

12. The pharmaceutical composition for use according to claim 1, wherein the insulinotropic peptide drug conjugate increases expression of Glut2 (Glucose transporter protein-2) involved in lipolysis.

## Patentansprüche

1. Pharmazeutische Zusammensetzung zur Verwendung bei der Prävention oder Behandlung einer nicht durch Alkohol bedingten Fettleber-Erkrankung, umfassend ein insulinotropes Peptid-Arzneimittel-Konjugat, hergestellt durch kovalentes Verbinden eines insulinotropen Peptids und einer Immunglobulin-Fc-Region über ein Nicht-Peptidyl-Polymer, als Wirkstoff, wobei das insulinotrope Peptid aus der aus Exendin-4, einem Exendin-4-Derivat, hergestellt durch Deletieren der N-terminalen Amingruppe von Exendin-4, einem Exendin-4-Derivat, hergestellt durch Substituieren der N-terminalen Amingruppe von Exendin-4 mit einer Hydroxylgruppe, einem Exendin-4-Derivat, hergestellt durch Modifizieren der N-terminalen Amingruppe von Exendin-4 mit einer Dimethylgruppe, und einem Exendin-4-Derivat, hergestellt durch Deletieren des α-Kohlenstoffs des N-terminalen Histidinrests von Exendin-4 und der N-terminalen, an den α-Kohlenstoff gebundenen Amingruppe, bestehenden Gruppe ausgewählt ist und
wobei das Nicht-Peptidyl-Polymer aus der aus Polyethylenglykol, Polypropylenglykol, Copolymeren von Ethylenglykol-Propylenglykol, polyoxyethylierten Polyolen, Polyvinylalkohol, Polysacchariden, Dextran, Polyvinylethylether, biologisch abbaubaren Polymeren, Lipidpolymeren, Chitinen, Hyaluronsäure und Kombinationen daraus bestehenden Gruppe ausgewählt ist, wobei das Nicht-Peptidyl-Polymer an einen Lysinrest an Position 27 des insulinotropen Peptids gebunden ist; und wobei die nicht durch Alkohol bedingte Fettleber-Erkrankung aus der aus einfacher Steatose, Fettleber-Erkrankungen, die durch Hungern ausgelöst werden, Leberfibrose und Leberzirrhose bestehenden Gruppe ausgewählt ist.

2. Pharmazeutische Zusammensetzung zur Verwendung nach Anspruch 1, wobei die Immunglobulin-Fc-Region bzw. das insulinotrope Peptid an beide Enden des Nicht-Peptidyl-Polymers gebunden sind.

3. Pharmazeutische Zusammensetzung zur Verwendung nach Anspruch 1, wobei das Nicht-Peptidylpolymer Polyethylenglykol ist.

4. Pharmazeutische Zusammensetzung zur Verwendung nach Anspruch 1, wobei die Immunglobulin-Fc-Region aglykosyliert ist.

5. Pharmazeutische Zusammensetzung zur Verwendung nach Anspruch 1, wobei die Immunglobulin-Fc-Region aus einer bis vier Domänen besteht, die aus der aus CH1-, CH2-, CH3- und CH4-Domänen bestehenden Gruppe ausgewählt sind.

6. Pharmazeutische Zusammensetzung zur Verwendung nach Anspruch 5, wobei die Immunglobulin-Fc-Region weiters eine Gelenkregion umfasst.

7. Pharmazeutische Zusammensetzung zur Verwendung nach Anspruch 1, wobei die Immunglobulin-Fc-Region eine Fc-Region ist, die von einem Immunglobulin abgeleitet ist, das aus der aus IgG, IgA, IgD, IgE und IgM bestehenden Gruppe ausgewählt ist, wobei die Immunglobulin-Fc-Region gegebenenfalls eine IgG4-Fc-Region ist und wobei die Immunglobulin-Fc-Region gegebenenfalls eine menschliche nichtglykosylierte IgG4-Fc-Region ist.

8. Pharmazeutische Zusammensetzung zur Verwendung nach Anspruch 1, wobei die reaktive Gruppe des Nicht-Peptidyl-Polymers aus der aus einer Aldehydgruppe, einer Propionaldehydgruppe, einer Butyraldehydgruppe, einer Maleinimidgruppe und einem Succinimidderivat bestehenden Gruppe ausgewählt ist.

9. Pharmazeutische Zusammensetzung zur Verwendung nach Anspruch 8, wobei das Succinimidderivat aus der aus Succinimidylpropionat, Succinimidylcarboxymethyl, Hydroxysuccinimidyl und Succinimidylcarbonat bestehenden Gruppe ausgewählt ist.

10. Pharmazeutische Zusammensetzung zur Verwendung nach Anspruch 1, wobei das Nicht-Peptidyl-Polymer reaktive Aldehydgruppen an beiden Enden davon aufweist.

11. Pharmazeutische Zusammensetzung zur Verwendung nach Anspruch 1, wobei das insulinotrope Peptid-Arzneimittel-Konjugat die Aktivität von PKC-ζ (Proteinkinase C-ζ) verstärkt, die die enzymatische Aktivität, die an der Lipolyse beteiligt ist, reguliert.

12. Pharmazeutische Zusammensetzung zur Verwendung nach Anspruch 1, wobei das insulinotrope Peptid-Arzneimittel-Konjugat die Expression von Glut2 (Glucosetransporterprotein-2) verstärkt, das an der Lipolyse beteiligt ist.

## Revendications

1. Composition pharmaceutique pour utilisation dans la prévention ou le traitement de la stéatose hépatique non alcoolique comprenant un conjugué peptide insulinotrope-médicament préparé par liaison covalente d'un peptide insulinotrope et d'une région Fc d'immunoglobuline par l'intermédiaire d'un polymère non-peptidyle en tant que substance active, dans laquelle le peptide insulinotrope est choisi dans le groupe constitué de l'exendine 4, d'un dérivé d'exendine 4 préparé par délétion du groupe amine N-terminal d'exendine 4, d'un dérivé d'exendine 4 préparé par substitution du groupe amine N-terminal d'exendine 4 par un groupe hydroxyle, d'un dérivé d'exendine 4 préparé par modification du groupe amine N-terminal d'exendine 4 avec un groupe diméthyle et d'un dérivé d'exendine 4 préparé par délétion du carbone alpha du résidu histidine N-terminal d'exendine 4 et du groupe amine N-terminal lié au carbone alpha, et le polymère non-peptidyle est choisi dans le groupe constitué du polyéthylène glycol, du polypropylène glycol, de copolymères d'éthylène glycol-propylène glycol, de polyols polyoxyéthylés, de l'alcool polyvinylique, de polysaccharides, du dextrane, de l'éther éthylique de polyvinyle, de polymères biodégradables, de polymères de lipides, de chitines, de l'acide hyaluronique, et des combinaisons de ceux-ci, dans laquelle le polymère non-peptidyle est lié à un résidu lysine à la position 27 du peptide insulinotrope ; et dans laquelle la stéatose hépatique non alcoolique est choisie dans le groupe constitué de la stéatose simple, des stéatoses hépatiques causées par l'inanition, de la fibrose hépatique et de la cirrhose du foie.

2. Composition pharmaceutique pour utilisation selon la revendication 1, dans laquelle la région Fc d'immunoglobuline et le peptide insulinotrope sont liés aux deux extrémités du polymère non-peptidyle, respectivement.

3. Composition pharmaceutique pour utilisation selon la revendication 1, dans laquelle le polymère non-peptidyle est le polyéthylène glycol.

4. Composition pharmaceutique pour utilisation selon la revendication 1, dans laquelle la région Fc d'immunoglobuline est aglycosylée.

5. Composition pharmaceutique pour utilisation selon la revendication 1, dans laquelle la région Fc d'immunoglobuline est composée d'un à quatre domaines choisis dans le groupe constitué de domaines CH1, CH2, CH3 et CH4.

6. Composition pharmaceutique pour utilisation selon la revendication 5, dans laquelle la région Fc d'immunoglobuline comprend en outre une région de charnière.

7. Composition pharmaceutique pour utilisation selon la revendication 1, dans laquelle la région Fc d'immunoglobuline est une région Fc qui est dérivée d'une immunoglobuline choisie dans le groupe constitué d'IgG, IgA, IgD, IgE et IgM, facultativement dans laquelle la région Fc d'immunoglobuline est une région Fc d'IgG4, et facultativement dans laquelle la région Fc d'immunoglobuline est une région Fc d'IgG4 humaine non glycosylée.

8. Composition pharmaceutique pour utilisation selon la revendication 1, dans laquelle le groupe réactif du polymère non-peptidyle est choisi dans le groupe constitué d'un groupe aldéhyde, d'un groupe propionaldéhyde, d'un groupe butyraldéhyde, d'un groupe maléimide et d'un dérivé de succinimide.

9. Composition pharmaceutique pour utilisation selon la revendication 8, dans laquelle le dérivé de succinimide est choisi dans le groupe constitué du propionate de succinimidyle, du succinimidylcarboxyméthyle, de l'hydroxysuccinimidyle et du carbonate de succinimidyle.

10. Composition pharmaceutique pour utilisation selon la revendication 1, dans laquelle le polymère non-peptidyle comporte des groupes aldéhyde réactifs aux deux extrémités de celui-ci.

11. Composition pharmaceutique pour utilisation selon la revendication 1, dans laquelle le conjugué peptide insulinotrope-médicament augmente l'activité de PKC-ζ (protéine kinase C-ζ) régulant l'activité enzymatique impliquée dans la lipolyse.

12. Composition pharmaceutique pour utilisation selon la revendication 1, dans laquelle le conjugué peptide insulinotrope-médicament augmente l'expression de Glut2 (protéine de transporteur de glucose 2) impliqué dans la lipolyse.


<110> HANMI SCIENCE CO., LTD.
<120> Pharmaceutical composition for the prevention or treatment of non-alcoholic fatty liver disease
<130> OPA13019/PCT
<150> KR10-2012-0024632
   <151> 2012-03-09
<160> 3
<170> KopatentIn 2.0
<210> 1
   <211> 31
   <212> PRT
   <213> Artificial Sequence
<220>
   <223> GLP-1
<400> 1
   His Ala Glu Gly Thr Phe Thr Ser Asp Val Ser Ser Tyr Leu Glu Gly
     1               5                  10                  15
   Gln Ala Ala Lys Glu Pro Ile Ala Trp Leu Val Lys Gly Arg Gly
                20                  25                  30
<210> 2
   <211> 39
   <212> PRT
   <213> Artificial Sequence
<220>
   <223> exendin-3
<400> 2
   His Ser Asp Gly Thr Phe Thr Ser Asp Leu Ser Lys Gln Met Glu Glu
     1               5                  10                  15
   Glu Ala Val Arg Leu Phe Ile Glu Trp Leu Lys Asn Gly Gly Pro Ser
                20                  25                  30
   Ser Gly Ala Pro Pro Pro Ser
            35
<210> 3
   <211> 39
   <212> PRT
   <213> Artificial Sequence
<220>
   <223> exendin-4
<400> 3
   His Gly Glu Gly Thr Phe Thr Ser Asp Leu Ser Lys Gln Met Glu Glu
     1               5                  10                  15
   Glu Ala Val Arg Leu Phe Ile Glu Trp Leu Lys Asn Gly Gly Pro Ser
                20                  25                  30
   Ser Gly Ala Pro Pro Pro Ser
            35